# EUROPEAN PATENT SPECIFICATION

(11) **EP 4 572 718 B1**
(45) Date of publication and mention of the grant of the patent: **21.10.2026**
(21) Application number: 23755376.3
(22) Date of filing: 11.08.2023
(51) Int. Cl.: A61F 6/06, A61B 5/00, A61B 5/01, A61B 5/145, H01Q 1/22, H01Q 1/27, H01Q 13/10, H01Q 21/24

(54) **VAGINAL RING DEVICE WITH BLUETOOTH CONNECTIVITY**
VAGINALRINGVORRICHTUNG MIT BLUETOOTH-KONNEKTIVITÄT
DISPOSITIF D'ANNEAU VAGINAL À CONNECTIVITÉ BLUETOOTH

(30) Priority: 18.08.2022 NL 2032800
(43) Date of publication of application: 25.06.2025
(73) Proprietor: Li Galli B.V., 2514 AC Den Haag (NL)
(72) Inventor: BENSON, Niels Jon, 2514 AC Den Haag (NL); RENNINGS, Andreas, 2514 AC Den Haag (NL)
(74) Representative: Arnold & Siedsma
(86) International application number: PCT/EP2023/072302
(87) International publication number: WO 2024/037980

(56) References cited:
- EP-B1- 3 359 099
- US-A1- 2006 084 848
- US-A1- 2012 276 854
- US-A1- 2015 282 763

## Description

The present invention is related to a vaginal ring, for instance a vaginal drug delivery device and/or diagnostic device, with wireless communication capabilities, in particular the capacity for Bluetooth connectivity, while positioned in the vagina.

Within the context of the present invention, a vaginal drug delivery device is a device configured for delivering drugs while inside the vagina. Similarly, a vaginal diagnostic device is configured for performing a diagnostic or measurement function while inside the vagina.

Vaginal drug delivery devices are known in the art. For instance, a low-dose contraceptive vaginal ring, known as Nuvaring^{®}, is manufactured from poly(ethylene-co-vinyl acetate). This ring releases hormones to provide contraception protection. However, it is noted that vaginal drug delivery devices have proven to be useful for delivery of some other types of drugs as well, due to their capability to release drugs where they can be directly taken up by the body, as well as their potential for gradually releasing drugs. Drug absorption can therefore be faster, avoid the hepatic first pass effect, and result in higher bioavailability. Vaginal drug delivery devices may further comprise sensors, either for controlling/monitoring the drug release, or for other diagnostic or monitoring functions.

Document US 2006/0084848 Al provides a device, and method and system for its use, for monitoring participants in clinical trials so that participant self-reporting, which is known to be notoriously inaccurate, can be minimized or eliminated. In preferred embodiments, the device is self-contained and self-powered, resides on or in a body cavity of the participant, collects data monitoring medically relevant aspects of the participant's behavior and of the local device environment, and stores data in a memory on-board the device. An accompanying external station reads stored data and prepares it for use. Devices may include electrically-active sensors and nonelectrical active sampling sensors. A preferred embodiment of the device is in clinical trials of microbicides inhibiting transmission of the HIV virus.

Document US 2012/0276854 Al discloses An implantable medical device may include a case which houses components of the implantable medical device. The implantable medical device may include an inductive coil coupled to a rechargeable battery. The inductive coil may be operative to inductively couple to an external coil and to transfer energy from the external coil to the rechargeable battery to recharge the rechargeable battery. The implantable medical device may include a cutout formed in the case of the implantable medical device and filled with a dielectric material. The cutout may be operative to reduce eddy currents in the case during recharge of the rechargeable battery. The implantable medical device may include a slot antenna disposed within the case. The slot antenna may be operative to communicate with an external device through the cutout in the case.

Such rings, regardless of intended application, generally have a substantially annular or oval shape, and are typically configured such that their shape can be temporarily transformed for insertion into the vagina, after which the original ring shape is regained, and the device is stably positioned at or near the Fornix Uteri or Cervix Uteri of a user. A particularly advantageous example of such a prior art is disclosed in EP 3359099 B1.

This prior art device comprises, in embodiments where the device is a vaginal drug delivery device, a reservoir for holding a medicament to be delivered, an outlet for dispensing the medicament, and a pump for pumping said medicament out of said outlet. In embodiments where the device is a diagnostic device, it comprises a diagnostic device for performing an intravaginal diagnosis or measurement therefore, for instance including one or more sensors. Combined devices which include the reservoir, outlet and pump, as well as one or more sensors, are also disclosed.

To allow the insertion, the device comprises two rigid members coupled at one end by a first flexible member, acting as a hinge, and at the other end by a flexible part; the first flexible member and/or the flexible part being configured for allowing the device to be squeezed by bringing the other ends together, thereby transforming the shape of the device from an extended shape, which corresponds to a substantially oval or annular ring shape, to a collapsed shape for allowing the device to be self-inserted into a vagina of a user at or near the fornix posterior vaginae. At least one of the first flexible member and flexible part is at least partially elastic in such a way that the device is pre-biased to assume the extended shape when no external force is applied.

It is already known, for instance from the above-mentioned document, to include a controller in such devices, generally in the form of a printed circuit board (PCB), for controlling the pump and/or diagnostic device.

The inclusion of a wireless communication device has also been considered, to communicate - generally via a controller - with such vaginal ring devices, for instance to obtain the data collected by a diagnostic device in the ring, to ascertain functioning of such a device, and/or to send commands to various elements in the vaginal ring device, in particular the pump, to control the delivery of the drug. Indeed, the above-mentioned document discloses the possibility to include a transmitter and/or receiver, for instance by incorporating a transceiver in the device, which may be placed in one of the rigid members.

However, wireless communication with a vaginal ring brings with it a number of significant challenges. The human body is a lossy medium for electromagnetic radiation to begin with, with a relatively high permittivity. When in use, a vaginal ring is surrounded by bodily tissue, of which the thickness can be significant in particular in individuals with a higher level of body fat in the area surrounding the vagina and uterus. Furthermore, one of the most convenient low-energy methods to communicate with a smart phone (or other types of personal electronic device with wireless capabilities) is generally using Bluetooth^{®}, which has a fairly short range even in ideal situations; in addition, the frequency band used for Bluetooth communication, ranging from 2.402GHz to 2.48 GHz, is quite sensitive to these effects. When radiating a signal to or from a vaginal ring situated in the body, significant losses of signal strength are observed, and there may also be issues keeping the frequency of the signal in-band. The constraints placed by body dimensions and user comfort on the size of the vaginal ring also place stringent limits on the size of any antenna or antenna array.

Therefore, while wireless communication with a vaginal ring device has been considered highly desirable for a number of years, and contrary to claims of some manufacturers, existing vaginal rings which incorporate a transceiver for wireless communication have not been able to achieve reliable, consistent wireless communication with a vaginal ring device inserted in the body, in a manner robust to body type variation, in particular not using Bluetooth signals, or other signals within (or close to) the Bluetooth frequency band. In fact, some existing devices (for instance temperature sensing devices used for fertility monitoring) which claim that they can be read out in use, in practise require users to extract the vaginal ring device each time they wish to read out the collected data. This is highly inconvenient, in particular for applications where up-to date measurements are ideally obtained with a relatively high frequency. For instance, for fertility monitoring, temperature is ideally measured at least once a day; other applications require a higher frequency than that.

Being able to communicate with a vaginal ring device while it remains inserted would be much more user-friendly. In addition to this, many other applications require the possibility for more frequent and direct communication, for instance highly time-sensitive diagnostic measurements, urgent commands, etc.

If such reliable, convenient communication is achieved, this also opens the way to many particularly advantageous advanced applications, including personalised and programmable drug delivery allowing complex schedules and bio signal based delivery, and even providing new insights by collecting precise, complete data from a larger group of patients.

Therefore, it is an object of the invention to provide a vaginal ring device capable, while inserted in the human body of wireless communication, preferably in the Bluetooth frequency band, which is reliable, consistent, and robust to body type variations.

In accordance with this object, a vaginal drug delivery and/or sensor device is provided, comprising a drug delivery unit comprising a reservoir holding a medicament to be delivered, an outlet, a pump for pumping said medicament out of said outlet, and a motor for actuating said pump; and/or a sensing unit comprising one or more sensors.

Such vaginal devices are typically deformable between an extended shape, which it assumes when little to no external force is applied thereto, and which corresponds to a substantially oval or annular ring shape extending around a central axial axis, and a collapsed shape allowing the device to be inserted into a vagina of a user. Here, the central axial axis is a theoretical axis around which the ring can be considered to revolve - since the extended shape is generally not a perfect torus, this axis may be defined in various ways, but it will have the same orientation as an axis of revolution for the smallest torus containing the device.

The proposed device further comprises a flexible printed circuit board, FPCB, comprising a first area and a second area. Control circuitry is arranged on the first area of the FPCB, communicatively coupled to the drug delivery unit and/or sensing unit. A wireless communication module is also arranged on the FPCB, preferably configured for Bluetooth communication, and which is communicatively coupled to the control circuitry. This wireless communication module may be arranged on the first area, on the second area, or on a further area of the FPCB. A slot antenna is formed on the second area of the FPCB by providing a slot in a metal layer arranged on the second area. A microstrip line connects the wireless communication module to a connection point at or adjacent to a first end of the slot. A ceramic buffer is arranged over the slot antenna on the second area; and at least one adhesive layer is arranged between the ceramic buffer and the slot antenna on the second area.

It has been found that through the combination of these features, the desired signal strength can be achieved within the Bluetooth frequency band.

Note that while the present application focuses on Bluetooth, the proposed device is not limited to the wireless communication module being a Bluetooth module. In particular, it is noted that wireless LAN communication / Wi-Fi (i.e. based on the IEEE 802.11 family of standards) can operate in the 2.4 GHz band as well (though it may require more power). The solution presented in this disclosure can therefore also be used to connect the device to a local area network, and/or to communicate to another device directly using peer-to-peer WLAN communication.

Generally, it is advantageous for the slot to be as long as possible, in particular longer than the second area, while radiating a signal in the desired frequency range. To that end, it is preferably for the slot to have a first end and a second end and to extend along a path between the first end and second end in such a way that the total length of the slot is significantly larger than the shortest distance between the first end point and second end point.

In particular, while some previous devices have been considered which include control circuitry as well as an antenna, it can be difficult to arrange these efficiently in the limited space available, especially in devices comprising a drug delivery unit. It is generally not possible to arrange all necessary electronic on a rigid printed circuit board within the space available. It would of course be possible to separately provide the control circuitry, wireless communication module, and (slot) antenna, and connecting these in various ways. However, such connections are vulnerable, and generally have a deleterious effect on the signal strength and characteristics. Therefore, a flexible printed circuit board, which makes it possible to provide various electronic elements (and in particular the antenna as well) on a single PCB while still allowing a compact arrangement, is advantageous. Preferably, the first area and second area of the FPCB are connected via at least one bent area, which makes it possible for the two areas to have a different orientation and/or arrangement. In many embodiments, the first area, which supports the control electronics, will be substantially planar, and will be arranged at an angle to at least part of the second area (11), which may also be substantially planar but does not need to be. For instance, by arranging the first and second areas to be substantially perpendicular, they can be arranged in line with a section of the outer wall of the device, while leaving space for other components.

In such a way, various competing constraints can be met: the bent arrangement of the FPCB makes it possible to increase the available area for the control circuitry and antenna, as well as the other electronic components such as the wireless connection module, while still arranging the various elements close to one another within the limited space of the housing. Furthermore, since the first area and second area are connected via at least one bent area, the length of required connection elements, most importantly that of the microstrip line, can be minimized to minimize losses between the communication module and the antenna; since the connections can be arranged on the FPCB, the resulting arrangement is also more robust.

The motor tends to be one of the bigger elements that need to be accommodated in the housing, and in advantageous embodiments which include a drug delivery unit (and, hence, a motor) the motor is at least in part arranged in a space delimited by the first area of the FPCB and the second area of the FPCB, or in other words: the FPCB is arranged around the (central axis of the) motor, which is advantageously compact, since it uses the space between the motor and the respective part of the housing.

Various considerations go into the specific orientation of the first and second areas. To achieve the desired directionality, it could be advantageous to position the second area, i.e. the antenna area, closest to the inner circumference of the ring. This is because, once positioned, the part of the ring which contains the various electronic elements, and which is generally thicker, is positioned further towards the user's back, and hence an antenna positioned closer to the inner circumference would radiate in a roughly forward direction, towards the user's pelvis area. The first area would then be positioned closer to the top or bottom of the ring, wherein the "top" and "bottom" are the parts that would be oriented, in use, substantially upward and downward, respectively. Looking purely from the perspective of the antenna, such an arrangement would likely be preferred. However, the inner circumference of the ring may not be available, in particular in view of the limited options to arrange rigid, longitudinal components such as a motor.

It was found that even if the second area, and thus the antenna, is arranged in a plane substantially perpendicular to the central axial axis, a suitable directionality can be achieved, in particular in combination with the proposed ceramic buffer and adhesive layer, and in combination with the use of an FPCB. Specifically, it was found that arranging the second area such that, when the device is in use, the second area is at the "top" side of the device, i.e. at the side oriented toughly upward, sufficient connectivity can be achieved with a phone positioned at various positions around the body. Note that since vaginal rings are often symmetric with respect to a plane which includes the central axis, and can therefore be inserted with either side up, it may be useful to add visual indications to the outside of the ring to make clear to a user what the preferred orientation is.

In embodiments in which the first and second area are substantially planar the first area would then generally be oriented substantially along a portion of the outer circumference of the device, preferably with the control circuitry and the communication module arranged on the side of the first area of the FPCB which faces the nearest portion of the outer surface of the device, for reasons of both compactness and performance. In other words: the surface of the first area of the FPCB on which the various electronic elements are arranged faces away, in embodiments in which the FPCB is arranged around the motor, from this motor.

The slot antenna can be arranged on the side of the FPCB which is oriented towards the ceramic buffer. In other words: in embodiments in which the FPCB is arranged around the motor, the surface with the slot antenna will be facing away from the motor. In cases where the second area is substantially planar, it was found that the radiation characteristics are quite similar towards both sides of the plane, and therefore the slot antenna could also be oriented away from the ceramic buffer, and therefore - in embodiment in which the FPCB is arranged away from the motor - towards the motor. A potential advantage of this may be that there is no direct contact between the metal coating and the at least one adhesive layer, which may increase the freedom in selecting the adhesive material; however, this could also be a disadvantage in some cases.

The second area may take the form of a slide flap of the FPCB. The first area, which generally will be larger than the second area, is then the "main" area of the FPCB. The advantage is that the FPCB may then be manufactured flat, with the side flap on which the slot antenna is formed being bent to accommodate the FPCB in the housing.

As discussed above, however, the second area in particular need not be planar, and embodiments could be conceived in which the second area is curved in accordance with the outer wall of the device.

Note that in practical applications, the FPCB will likely include more than two areas, which can have various relative orientations, for instance to improve connection to a battery of the device, to the motor and/or pump, and/or to any sensors that may be present. For instance, if the second area takes the form of a slide flap of the FPCB, the FPCB may further include a second side flap which includes terminals for the battery/power source. This side flap may then be bent so that it is arranged perpendicular to both the first area and the second area. Alternatively, or additionally, a side flap may be provided which provides the required communicative coupling between the control circuitry and the motor. Further electronic components may also be provided, and may be arranged on the first area, second area, or any potential further area.

The use of a slot antenna makes it possible to arrange a relatively long antenna, which is required for the Bluetooth frequency, in a very limited space. Generally, the metal layer is a copper and gold coating, or other suitable metal coating, applied to the second area. In particular, it has been found advantageous to use a slot antenna which has a single, continuous slot which takes a meandering, zigzagging or otherwise twisting and/or three-dimensional path between its first end point and its second end point, such that its total length can be (much) longer than the shortest distance between these two points. Such an antenna is a magnetic antenna with reduced electric components in the near field.

It is however not excluded that examples with two or more separate slots could also be used. The use of multiple slot antennas may increase the signal and can furthermore be compatible with multiple input-multiple output applications. These slots could both/all be arranged on a same area of the FPCB, but could also be arranged on several areas; note that in case they are distributed over more than one area, more than one ceramic buffer may need to be provided, and accordingly also at least one additional adhesive layer. As an example, one disadvantage of having a single, continuous slot is that it will radiate a signal with a certain polarity. As a result, for certain phones (or other devices with Bluetooth connectivity) the connectivity with a phone may depend on the relative orientation of (the Bluetooth antenna in) the phone. By providing two slots, arranged such that they radiate signals with mutually orthogonal polarity, this can be avoided. On the other hand, such solutions require more space, which may not be available.

To radiate in the correct frequency band, the radiating length of the slot, i.e., the total length of the path starting at the connection point, should be an integer multiple of the half-wavelength, i.e. *n* λ/2.; as with other types of antennas, integer fractions, like λ/4 or 5/4 λ, may also be considered.

In embodiments, the second area is substantially rectangular, with two shorter sides and two longer sides, wherein the first end of the slot is located close to a first one of the shorter sides, and the second end of the slot is located close to the other of the shorter sides. The second area may then be connected to the first area over at least a portion of a first one of the longer sides; however, it could also be considered to orient it with one of the shorter sides arranged along a side of the first area. For instance, two or three bridging portions may be provided. The second area could also be connected to the first area over the entirety of one of its longer sides, but this is generally not necessary, and may add unnecessary bulk. In such a rectangular second area, the slot preferably follows a meandering path comprising longer sections extending substantially parallel to the width of the second area; and shorter sections extending substantially parallel to the length of the second area, which connect one of the ends of adjacent longer portions. This generally allows for the longest path despite the limited area. However, alternatives could of course be considered, for instance having only a few, but longer, meanders; as well as using paths along more than two directions, turns which are not at right angles, and even curved path sections. Generally, to avoid potential irregularities, for instance due to interference, it is recommended - regardless of the specific shape and dimensions of the path - to keep the distance between adjacent sections of the path comparable or larger than the width of the slot itself. In the specific case above, this corresponds to having the distance between two adjacent longer sections at least as big as the width of the slot.

As described above, a microstrip line connects the wireless communication module to a connection point at or adjacent to the first end or second end of the slot. "Adjacent", here, may be interpreted in such a way that the distance between the first end and the connection point is comparable to the shortest distance between the first end and the central longitudinal axis of the slot. This is to improve radiation efficiency, since the maximum field is at the center of the slot. The slot however advantageously extends slightly beyond the connection point, wherein this extended portion can be configured to improve impedance matching between the wireless communication module and the slot. For impedance matching, such a microstrip is generally configured to have a resistance of about 50Ω, specifically where the real part is 50Ω (the imaginary part being 0Ω). The most convenient way to do so tends to be to choose the width appropriately, since the length of the microstrip line tends to be selected to maximize the potential difference between the point at which it connects to the wireless connection module and the connection point where it connects to the slot. To avoid undesirable interference effects, it can be useful to arrange a substantial section of the microstrip line on the opposite side of the second area of the FPCB than the slot antenna. One end of the microstrip line (i.e. the end which should be connected at the connection point) then passes through an opening in the second area at or close to the connection point. If the wireless connection module is arranged on the same surface of the FPCB as the antenna, another opening will then need to be provided in the first area to arrange the connection to the wireless connection module. In case the wireless connection module is arranged in the first area, the microstrip line will run via a bent portion connecting the first and second areas; however, the wireless connection module could also be arranged on the second area, in which case this is not needed.

One big issue in communication with a device located in the human body is that body tissue is a lossy medium, and there can be significant near-field losses. Slot antennas are an advantageous choice in this context because they have weak electric nearfields; however, this by itself has proven not to be sufficient, in particular for slot antennas with such stringent size constraints.

To further reduce the losses, a ceramic buffer is arranged over the slot antenna on the second area. While the use of these ceramic buffers has previously been considered, its advantages in this particular context and applications have been found to be particularly pronounced. In particular, while such a buffer may already have some positive effects on the radiation outside the body, these improvements are even more pronounced when the device is inserted, to such an extent that it makes it possible to minimize the size of the antenna to fit into the limited space in the ring.

The ceramic buffer may have various shapes and sizes; however, it preferably has a first major surface which covers the slot antenna, wherein the first major surface is preferably substantially parallel to the surface of the second area, so that the distance between the second area and the first major surface is substantially constant. The first major surface may have a larger area than the second area, or may have the same area; it is preferable that at least all parts of the second area on which the slot antenna is arranged are covered by the ceramic buffer, so that it can in a sense "trap" the near field.

Generally, it was found that results are improved if the ceramic buffer is configured such that it substantially fills the space between the slot antenna and the nearest portion of the outer wall of the device, i.e. the portion of the outer wall which, when seen along the central axial axis, is located over the slot antenna. In other words: the ceramic buffer is advantageously shaped and sized so that the first major surface is defined in dependence on the size, shape and location of the second area, and the opposite surface is shaped to conform to the inside of the housing. This also ensures that there is little to no air between the radiating surface of the slot antenna and the housing, which further helps reduce losses and improve impedance matching with the surrounding tissue.

It could even be considered to have this surface of the ceramic buffer forming part of the surface of the device itself, or perhaps even to manufacture at least part of the housing from a medically acceptable ceramic or ceramic-containing material.

Zirconia has proven to be an advantageous choice of material for the ceramic material. It is preferable for the ceramic buffer to have a relative permittivity εᵣ between 10 and 50, in particular between 20 and 40, or even more preferably between 25 and 35. This is related to the fact that the relative permittivity of the human body is generally between 40 and 60.

Due to the fact that many elements are arranged in a small space, the strength, directivity, and even frequency of the emitted radiation are influenced by various factors. Due to this, it cannot be assumed that antenna characteristics measured outside of the device and outside of the body will be maintained. For instance, different values for the operational frequency and for the matching level were found depending on the presence or absence of the buffer; the arrangement with respect to the motor; the bending of the FPCB, and even the material of the housing. These effects in some cases led to the frequency no longer being in the required frequency band for Bluetooth communication.

In the device, at least one adhesive layer is present, primarily to attach the first major surface ceramic buffer securely to the surface of the second area of the FPCB, and thus over the slot antenna. Surprisingly, the thickness and material characteristics of this adhesive layer were also found to influence the radiation characteristics. In particular, while many of the above-mentioned elements resulted in the antenna operation frequency shifting to a lower value, it was found that by carefully selecting the thickness of the at least one adhesive layer, at least part of this shift could be compensated for.

Note that in some cases, a similar effect could perhaps be achieved by increasing the distance between the second area of the FPCB and the first major surface of the ceramic buffer in some other way. However, in comparing the results with a suitably chosen adhesive layer (or layers) to results when mechanical spacers are used to ensure a same or similar distance between the second area of the FPCB and the first major surface of the ceramic buffer, surprising improvements have been observed.

It was found to be preferably for the at least one adhesive layer to have a thickness between 50 µm and 200 µm, preferably between 80 µm and 170 µm, more preferably between 90µm and 120µm.

Furthermore, it was found that the radiation performance of the proposed ring in some cases degraded over time. However, by correctly selecting the thickness and/or material properties of the at least one adhesive layer, this degradation could also be limited. Particularly advantageous results were found when the at least one adhesive layer was a multi-layer structure; for instance comprising a carrier layer between two adhesive layers. This carrier layer is preferably a polyester layer, and the two adhesive layers are preferably modified acrylic adhesive layers; however, the advantageous effects do not seem limited to these particular material choices.

Generally, the dimensions of the antenna pattern, the size of the ceramic buffer, and the arrangement of the FPCB as a whole inside the device are highly limited by the small dimensions required to allow for insertion in the vagina and comfortable arrangement in the body. This limits the configurability of these elements. However, there is some more freedom, even at a relatively late stage in the design process, in selecting the thickness and/or material properties of the at least one adhesive layer, and in advantageous embodiments this makes it possible to compensate for the effect of the ceramic buffer, motor, bent FPCB and/or or housing on the operation frequency, so as to ensure that the signal emitted by the device is in the Bluetooth frequency band. Furthermore, this may aid in optimizing the matching level, and prevent degradation of the signal properties over time.

In embodiments, the device further comprises a first rigid member having a first and second end; a second rigid member having a third and fourth end; a first flexible member coupled between the first and third ends; and a flexible part coupled between the second and fourth ends. In such a device, the reservoir may then be accommodated in the first rigid member; the outlet may be provided in the outer wall of the second rigid member; the pump and motor may be accommodated in the second rigid member; and the device further includes a tube which passes through the first flexible member and provides a fluid communication path between the reservoir and the outlet. If the pump is a roller pump, it is preferably arranged to push fluid through the tube to the outlet.

However, the elements described above can also be implemented in other types of vaginal ring devices.

In particular, it is noted that the present application focuses on embodiments including a drug delivery unit, since these tend to pose the most challenges: the sizes of the motor and pump can only be minimized to a certain extent, and the size of the reservoir should be sufficient so that the medicament does not need to be frequently replenished. For embodiments with only a sensing unit, and no drug delivery unit, the constraints may, depending on the selected sensors, be less stringent at least in some respects; in particular, the various electronic elements (including any sensors) can in such embodiments also be arranged elsewhere in the device. However, even in such embodiments achieving the desired radiation characteristics has proven challenging, and the principles outlined in this application may be advantageously applied.

The device may also comprise a power source, such as a battery, preferably accommodated in the second rigid member. This battery may provide power to the FPCB, as well as the motor (if present) and/or other elements. The battery may be arranged so that its terminals contact terminals on a further area of the FPCB, which may be configured as a side flap. The motor is advantageously positioned between the power source and the pump. The at least one sensor, if present, may be arranged in the first and/or second rigid member, though it is noted that some types of sensors could be arranged at least in part in a flexible section of the device.

The invention will be further elucidated at the hand of the figures, wherein:
Fig. 1 illustrates the insertion and positioning of a typical vaginal ring device;
Fig. 2A and 2B show an example vaginal ring device in its extended and collapsed state, respectively;
Fig. 3 is a perspective view of an embodiment of the vaginal ring device according to the invention, with the top part of the first and second rigid members shown separate from the remainder of the device;
Fig. 4 is a perspective view of an embodiment with the top part of the member in which the FPCB is arranged removed;
Fig. 5 is a top view of the embodiment of Fig. 4;
Fig. 6 shows a cross-section of the part of the vaginal ring device in which the FPCB is arranged along a radial direction;
Fig. 7A and 7B are perspective views showing the inside of the part of the rigid member in which the FPCB is arranged;
Fig. 8A shows a ceramic buffer in accordance with the invention, arranged on the second area of an FPCB; Fig. 8B shows another ceramic buffer;
Fig. 9A and 9B schematically illustrate two possible embodiments of a second area in accordance with the invention;
Fig. 10 schematically shows an example of a flexible circuit board in accordance with the invention.

In the following, similar elements are indicated using the same reference numbers; unless indicated otherwise, the description of an element in the context of one figure is also applicable to elements in other figures with the same reference number.

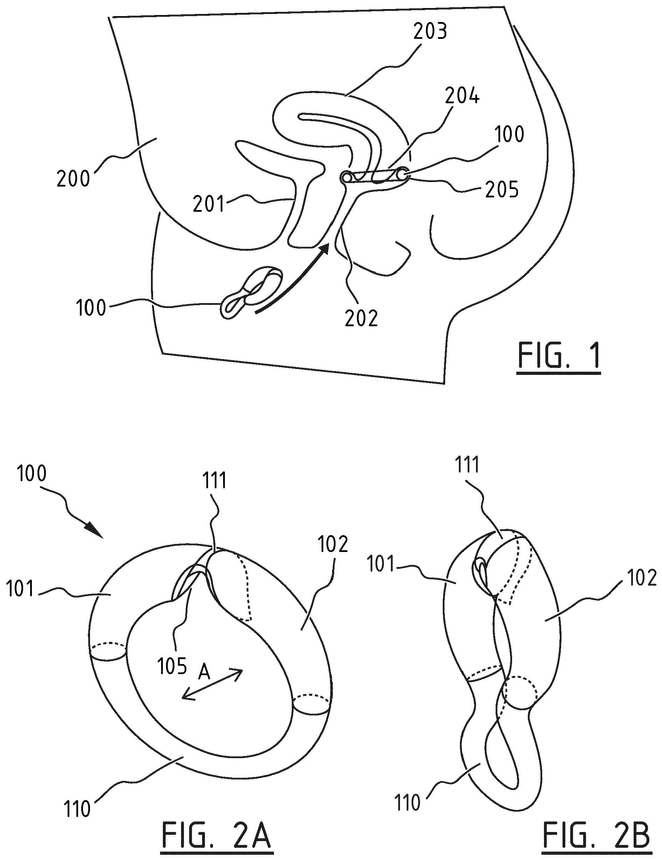

Fig. 1 illustrates the insertion and positioning in use of a known vaginal ring device 100; such a device is shown in its expanded and its collapsed state in Fig. 2A and Fig. 2B, respectively.

In Fig. 1, a body 200 of the user is shown indicating the urethra 201, the vagina 202, and the uterus 203. Prior to inserting device 100, it is squeezed to transform the shape into the collapsed state. Thereafter, the user inserts device 100 into vagina 202, and moves device 100 close to Cervix Uteri 204 or Fornix Uteri 205, where device 100 is released. Due to its elasticity, device 100 regains its original shape, at least to a substantial extent. Due to its shape and size, device 100 rests against the vaginal wall. This positioning allows localised, gradual drug release, as well as appropriate measurements to be performed. Moreover, this position ensures that little to no pain is observed by the user and that any impact of device 100 on coitus is minimized.

Fig. 2A and 2B show the device in more detail, in both the expanded state it is biased towards, and reverts to once positioned, and in the collapsed state for insertion.

This shape comprises a first rigid member 101, a second rigid member 102, a first flexible member 111, and a flexible part 110. Here, first flexible member 111 is made of an elastic material and comprises a recess 105 to allow first and second rigid member 101, 102 to move towards each other when device 100 is squeezed into the collapsed state. The flexible member 111 and/or flexible part 110 are pre-biased to that the device will assume its extended shape (again) when little to no external force is applied thereto. In the extended shape, the device has a generally oval or annular ring shape extending around central axial axis A. For the purposes of the description below, the precise location of this (theoretical) axis A is not particularly relevant, but its orientation may be used to define the "top" and "bottom" of the device.

According to the invention, the functional parts of the drug delivery and/or diagnostic mechanism are preferably incorporated into the rigid members 101, 102, whereas any necessary electronic and/or fluidic connections between these functional parts can be accommodated in the flexible member 111 or flexible part 110. However, some functional components may also be accommodated in flexible member 111 and/or flexible part 110. For instance, it is not excluded that (part of) one or more sensors may advantageously be accommodated in or as part of the flexible member 111 and/or flexible part 110; or that the flexible member 111 and/or flexible part 110 may be configured to advantageously influence the radiated signal. In other examples, flexible part 110 may be composed of multiple parts, some of which may be rigid.

It is noted that the invention is not limited to this particular vaginal ring device, and that the principles outlined herein can also be applied to various other or still to be developed vaginal ring devices. However, all these devices have similar limitations, in particular that many of the electronic components need to be accommodated in a rigid part or parts, but that such rigid parts are limited in size and shape by the required collapsibility. The outer diameter of the device, determined in a plane perpendicular to axis A, is generally between 50 and 70 mm, more preferably between 55 and 65 mm, and the internal diameter, determined in a cross section parallel to axis A, is typically between 4 and 8 mm. In view of this, the size of all components is highly limited.

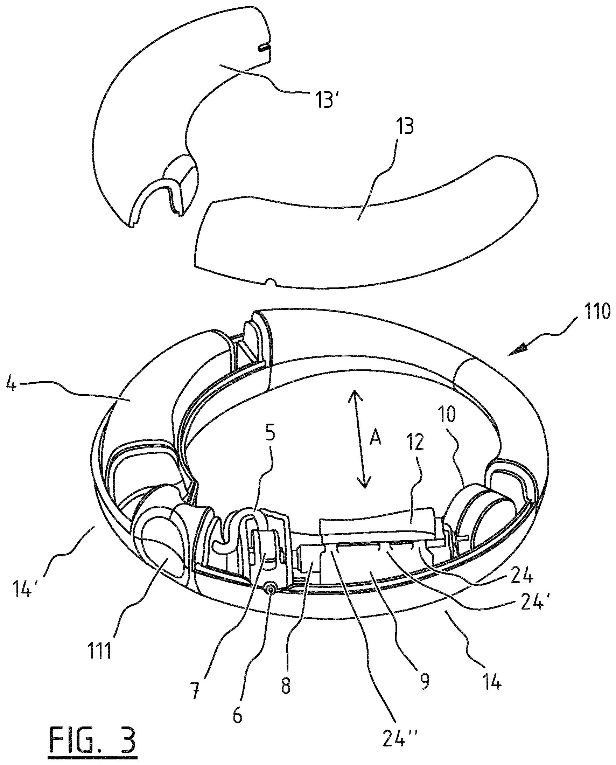

Fig. 3 is a perspective view of an example vaginal ring device. This device comprises a drug delivery unit; though it is not illustrated, it may also comprise a sensing unit comprising one or more sensors. In the depicted embodiment, first rigid member 101 and second rigid member 102 are each formed by a top housing part 13, 13' and bottom housing part 14, 14' which can be attached to one another in a water-proof manner; however, it can be envisaged to form the rigid members in a different manner, for instance as an integrally formed tube into which components can be inserted. To illustrate the arrangement of the various components, in Fig. 3 the top housing parts 13 and 13' are shown as separated from the device. In some embodiments, this is representative of a stage of the assembly process; however it may also be that the top and bottom housing parts are assembled to form the rigid members prior to the assembly of rigid members 101, 102 with flexible part 110 and flexible member 111. Some or all elements may also be manufactured through injection moulding and/or 3D printing. Though not shown, these top housing parts 13, 13' and bottom housing parts 14, 14' may include means, on their inner surface, to position and/or support the various components.

It should be clear that for all discussed embodiments, a mirrored arrangement is also possible, in particular in embodiments such as the illustrated embodiment for which rigid members 101, 102 are substantially similar in size and symmetric in shape.

The drug delivery unit comprises a reservoir 4 holding a medicament to be delivered which is accommodated in second rigid member 102; in this embodiment, an outlet 6 for dispensing the medicament is provided in the outer surface of first rigid member 102, specifically along the joint between top housing part 13 and bottom housing part 14; however, it could also be provided elsewhere. Since in this embodiment reservoir 4 and outlet 6 are in different rigid members, tube 5, which provides the fluidic connection between reservoir 4 and outlet 6, passes through flexible member 11; the tube is therefore also flexible. In this embodiment, the dispensing of medication is controller using roller pump 7, which is located close to the outlet and can be controlled to push the liquid active ingredient - located inside the tube - through the tube and outwards into the vaginal cavity at the tube outlet. However, other types of pumps could also be used, in which case it may also be possible to accommodate the pump in the rigid member which accommodates reservoir 4, instead. Outlet 6 and roller pump 7 are preferably positioned fairly close to flexible member 111, since this minimized the required length of tubing.

Note that the particulars of the drug delivery unit are not the focus of the present application, and that this unit will typically include further elements, not depicted in these figures. So instance, the rigid member in which reservoir 4 is arranged typically also includes a valve, for instance an anti-vacuum pressure valve, to compensate for the changes in pressure caused when emptying the reservoir to dispense the medicament, and to prevent liquid from the vaginal cavity from entering the device. A filling hole may also be provided in the rigid member in which reservoir 4 is arranged, to allow reservoir to be (re)filled.

To drive the pump, motor 8 is also arranged in first rigid member 101, with its longitudinal axis generally oriented along the toroidal direction of the ring, i.e. perpendicular to a radius. Battery 10 is arranged in first rigid member 101 as well, in order to provide power to both motor 8 and the control electronics on first area 9 of FPCB 30.

First area 9 of FPCB 30 is, in this embodiment, arranged facing towards the outer circumference of ring device 100. FPCB is bent in this embodiment over three bending areas 24, 24'and 24" connecting first area 9 to second area 11 (not visible in Fig. 3), on which the slot antenna is provided. As a result, second area 11 is substantially perpendicular to first area 9. Due to this bending, FPCB 30 can be arranged in a very compact manner, around motor 8, in the space between motor 8 and the housing. Ceramic buffer 12 is positioned over second area 11, specifically so as to fill most of the space between second area 11 and the section of top housing part 13 located above second area 11.

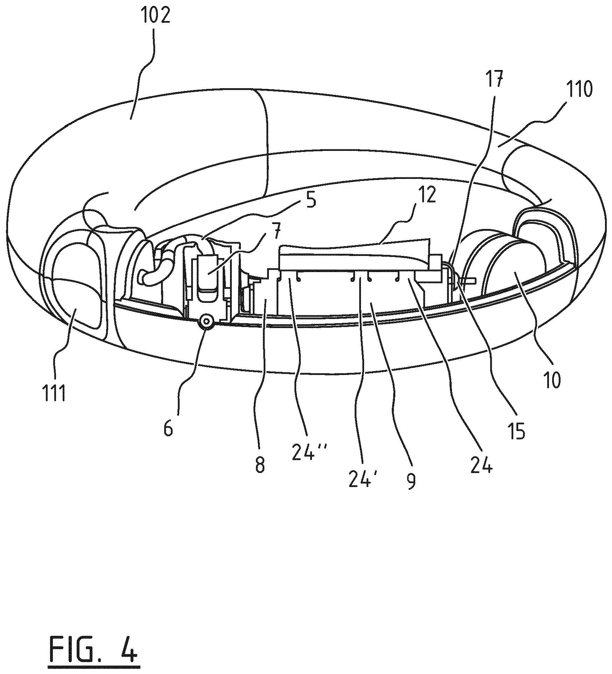

Fig. 4 is a different view of the embodiment of Fig. 3, with top housing part 13 of first rigid member 101 omitted. On this figure, it can be seen that in this embodiment, the FPCB also includes a further side flap, connected in this case to second area 11, which provides a connection to a terminal 15 of motor 8. Other ways of connecting motor 8 to FPCB 30 and motor 8 can however also be used. Though not shown in this figure, the FPCB also includes further sections/areas which can be arranged to connect to both terminals of battery 10, to ensure power supply both to the various electronic components on the FPCB and to motor 8.

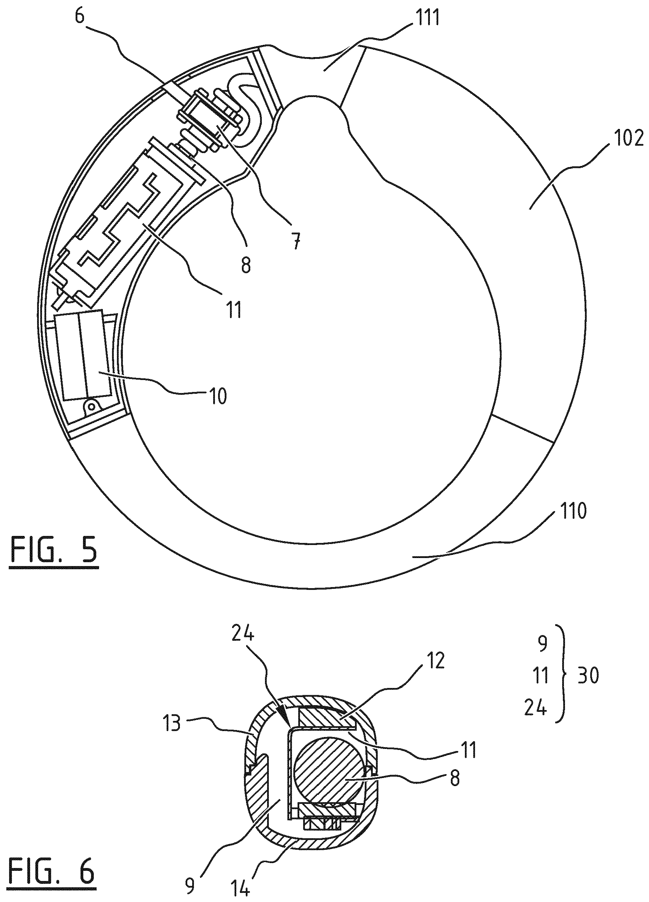

Fig. 5 shows a top view of the same embodiment, in which not only top housing part 13 but also ceramic buffer 12 have been omitted, in order to show the arrangement of the slot antenna on second area 11. Note that the shape of the slot antenna in this figure is schematic and not intended to illustrate a specific path shape.

Fig. 6 is a schematic cross section of first rigid member 101, showing the arrangement of first area 9 and second area 11 of the FPCB, connected by bending area 24, around motor 8. This figure also illustrate one possible manner of attaching top housing part 13 to bottom housing part 14 in a secure manner. Furthermore, in this figure, it can be seen that ceramic buffer 12 is configured so that there is little to no empty space between second area 11, on which the slot antenna is provided, and the inner surface of top housing part 13.

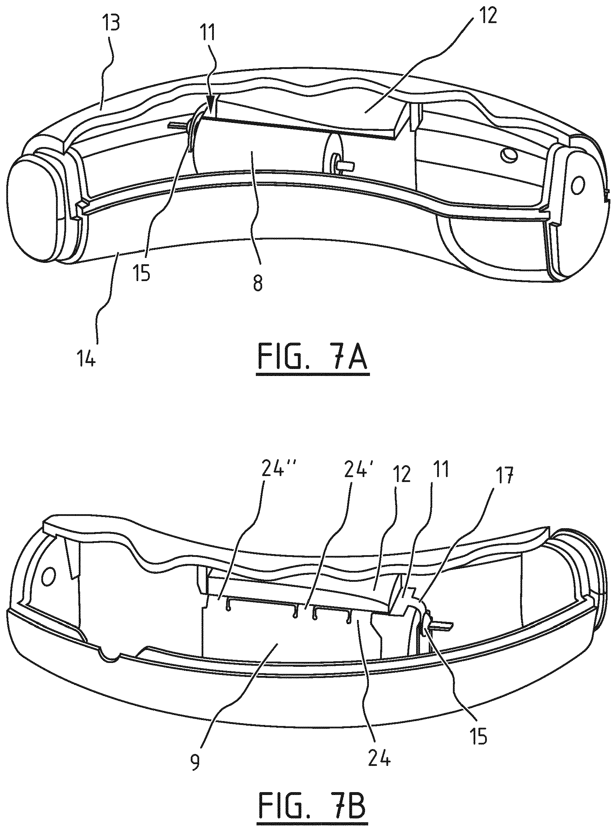

Fig. 7A and 7B show only the first rigid member, with several elements such as pump 7 and battery 10 omitted to more clearly illustrate the arrangement of FPCB 30 around motor 8. As can be seen, FPCB 30 is bent along bending areas 24, 24'and 24" so that first area 9 and second area 11 are substantially perpendicular to each other, and along a further bending area 17 to provide terminal 15. However, the angle need not be perpendicular; furthermore, the first area 9 may itself be embodied as including several sub-areas, which may not all be in the same plane, as illustrated below.

While the figures discussed above do not show a sensing unit, it will be clear to the skilled person that various sensors can be arranged in the shown device, in addition to or instead of the various elements of the drug delivery unit depicted in these figures. These sensors are then typically communicatively coupled to (at least) the control circuitry, so that sensor values measured by the sensor can be relayed, processed and/or analysed.

While sensing unit in vaginal devices can advantageously (also) be used for diagnostic purposes, the invention is not limited to such sensors, and the device may also include one or more sensors for internal regulation purposes.

For instance, in embodiments with a drug delivery unit, it may be useful to provide a temperature sensor (which need not be particularly precise), for instance arranged on the FPCB, simply to be able to check if the temperature of the device is close to the temperature of the human body, indicating that the vaginal device is currently inserted in the vagina. In such cases, the values measured by the sensor need not be transmitted to another devices, but the control circuitry could for instance be configured to interrupt drug delivery if the sensed temperature value indicates the device is not currently in the human body. In another example, a sensor could be provided which monitors the amount of medicament left in the reservoir. In such cases, the control circuitry may be configured to use the sensor information to confirm proper functioning of the drug delivery mechanism and/or to emit an alert (either via the wireless communication module or in some other way) if the reservoir is almost empty. A combination of a drug delivery unit and a sensing unit may also be used to provide a self-regulating feedback mechanism, wherein the measured level of an active ingredient of the medicament, or of a factor indicative of the current need for this medicament, is used to control the drug delivery mechanism. For instance, for diabetes patients, a blood glucose sensor could be used to regulate the release of insulin.

In the above cases, transmission of the sensor measurements, or values derived from these measurements to an external device by the wireless communication module, using the antenna, may not be required, but could still be useful. For instance, the temperature measurements above, which are indicative of whether the device is *in situ,* can be used to confirm compliance/drug adherence.

. Such transmission of measurements is of course particular advantageous if the sensing unit - which can be provided along with a drug delivery unit but also separately - includes diagnostic sensors/bio sensors. Monitoring body temperature (which may include analysing temperature fluctuations) for fertility monitoring was already mentioned above. Note that this would require a more accurate temperature sensor than the one described above, preferably arranged such that it is able to measure the temperature of the surrounding tissue while minimally affected by the heat produced by the various electronic components. To further improve the accuracy of fertility monitoring (including for *in vitro* fertilization) in addition to (or instead of) at least one temperature sensor, the device could include a biosensor measuring the level of luteinizing hormone (LH) and/or estradiol (E2). Such biosensors may require direct contact with the vaginal mucosa. To this end, the device may be provided with at least one opening and/or membrane if this is required to perform the relevant measurements. Such openings and/or membranes will be located at such places on the outer or inner perimeter of the ring to guarantee optimal contact with the vaginal mucosa or cervical mucus.

The diagnostic applications are however not restricted to those relating to fertility, or even to sex-specific issues more generally. The sensing units may include any type of sensor which can measure diagnostically relevant information while inserted in the human body. This may even include microfluidic sensors, preferably of the lab-on-a-chip type, capable of detecting various complex modules such as biomarkers in liquid obtained from the surroundings of the device - provided, of course, that these can be sufficiently miniaturized so that they fit within the device. Generally, it is preferable to use sensors with a maximal diameter of the order of 1 cm, and a thickness of no more than 5mm. Note that the analysis and processing required to interpret signals from such sensors can be performed by a processing module in the device; by at least one external device to which data is transmitted; or by suitably distributing the required computations between a module or modules provided in the device and at least one external device.

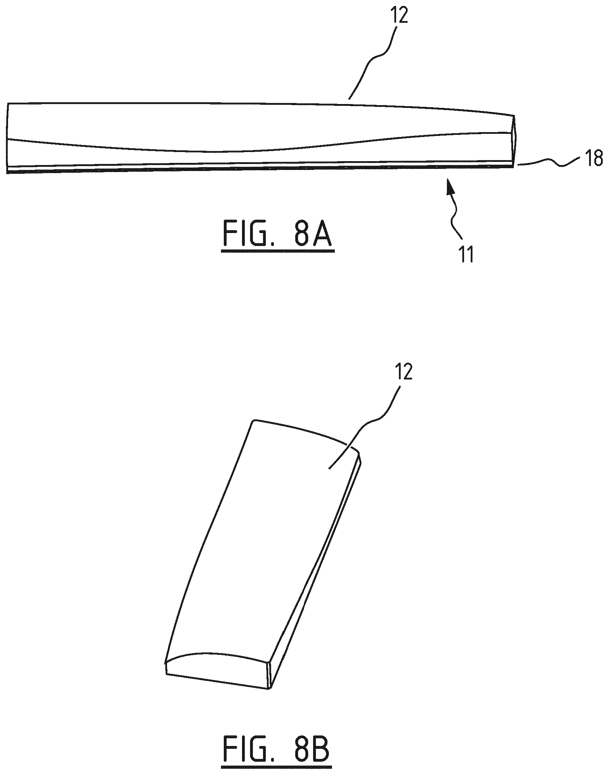

Fig. 8A and 8B show two example ceramic buffers 12. While the specific shapes are different, these each have a planar first major surface, which in Fig. 8A is shown to be attached to second area 11 via at least one adhesive layer 18. The other major surface is curved in accordance with the shape of the section of the inner surface of the rigid member in which the FPCB is arranged, so that the space between the slot antenna on second area 11 and this inner surface is substantially filled.

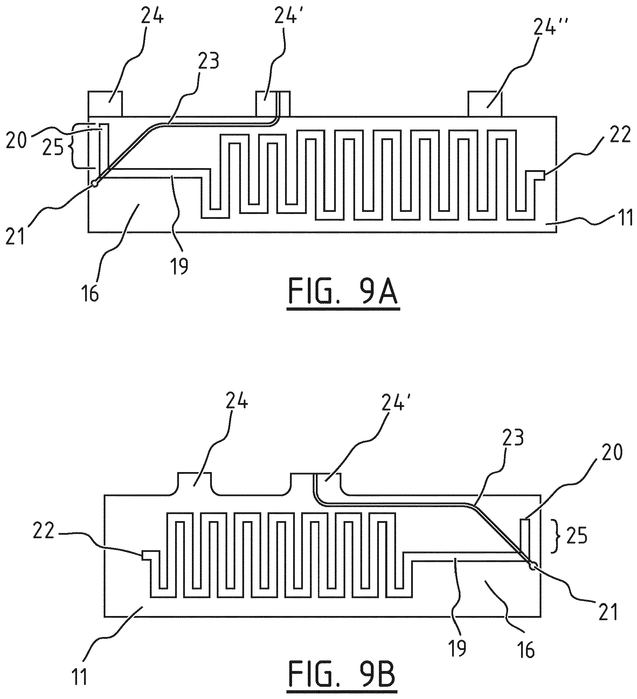

Fig. 9A and 9B illustrate two possible embodiments for the slot antenna, and in particular the slot 19, on second area 11. The slot antenna is provided as a slot 19 in a metal coating 16 on second area 11, i.e., as a path along which this coating is removed (or not provided). The slot antenna is provided as a single, elongated slot 19 which goes from a first end 20 to a second end 22 along a meandering or twisting path, in such a way that the total path length is substantially longer than the shortest distance between first end 20 and second end 22. This is because the length of the second area, due to the limited space available in such vaginal ring devices, is typically of the order of 1-2 cm, which is generally not sufficient for generating a Bluetooth signal, in particular one of sufficient strength. In the example shown in Fig. 9A and Fig. 9B, the path length from connection point 21 to second end 22 corresponds approximately to 2 λ, i.e. n λ/2 with n = 4, which yields - in combination with the other elements - a sufficient radiation strength within the space constraints in a device comprising a drug delivery unit. Remaining section of slot 25, between connection point 21 and first end 20, is selected to optimize the impedance matching with the wireless communication module/Bluetooth chip. First end 20 and second end 22 are typically at opposite ends of second area 11, though this may not in all cases be necessary. The width of the slot, which is preferably substantially constant along the length of the path, is generally of about 0.2 to 0.4 mm; to avoid interference, the distance between adjacent portions of the slot should be at least as large as this width.

In the embodiment of Fig. 9A, there are three bending areas 24, 24' and 24" connecting second area 11 to first area 9 of FPCB; in Fig. 9B, there are two bending areas 24, 24'. It is not excluded that first area 9 and second area 11 would be connected over the full length; however, using smaller, separate bending areas tends to facilitate the correct bending and positioning. Aside from this, one of the bending areas also provides a route for microstrip line 23. While microstrip line 23 is depicted in these figures, it is advantageously provided for the most part on the opposite surface to that where the slot antenna is provided, and would therefore not be visible. At connection point 23, a through-hole is then provided in second area 11. However, note that microstrip line 23 should in general not overlap with or cross slot 19, aside from close to connection point 21, in view of avoiding interference. In the embodiment of Fig. 9A, the path of slot 19, in particular the length of the sections which run vertically in the figure, is adapted to allow space for microstrip line 23; in the embodiment of Fig. 9B, this is not required. Microstrip line 23 ends at connection point 21, which is located close to a first end 20 of slot 19; as mentioned, while the section of slot 19 between connection point 21 and first end 20 does not affect the radiated signal directly, it is useful to improve impedance matching with the wireless communication module/Bluetooth chip to which it is connected via microstrip line 23, and its length is preferably selected in accordance with this.

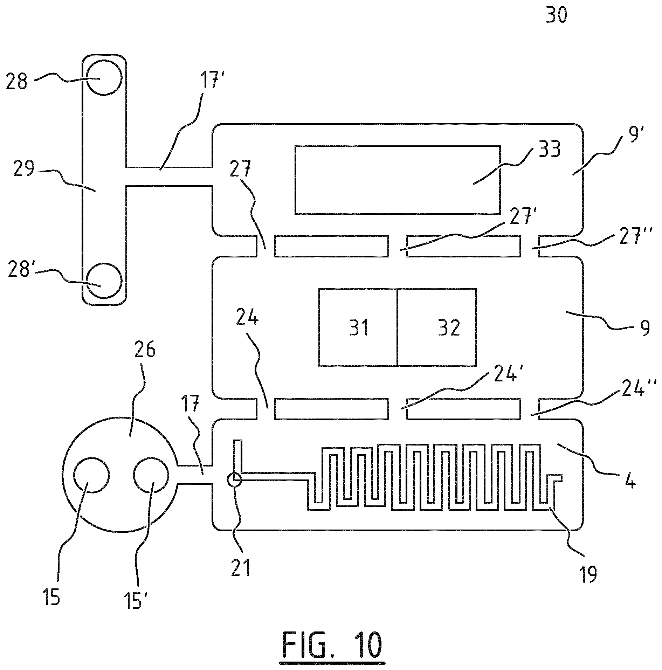

Fig. 10 shows a schematic illustration of a FPCB 30 in accordance with the invention, in particular prior to its bending and arranging in a device, i.e., arranged in a plane. In this figure, the antenna of Fig. 9A is schematically depicted, but of course the invention is not limited to this particular antenna shape. Second area 11 is connected to first area 9 via bending areas 24, 24'and 24" - fewer or more bending areas could also be used. Second area 11 is also connected to motor connection area 26 via bending area 17; this further area 26 includes terminals 15 and 15' for connection to the motor.

In this embodiment, the FPCB has a third area 9', which is connected to first area 9 via further bending areas 27, 27' and 27", and which is arranged on the opposite side of first area 9 than second area 11. As a result, the FPCB can be bent at bending areas 24, 24' and 24" as well as bending areas 27, 27' and 27" in such a way that the FPCB can be arranged to partially surround a component arranged along the circumference of the device, such as motor 8 depicted in some of the other figures. Note that area 9' could also be seen as a sub-area of the first area, with the first area then formed by the combination of areas 9 and 9' - generally, it is not necessary for the first area to be arranged in a single plane in the device, nor for this first area to comprise all of the control circuitry.

In Fig. 10, main processing unit 31 and wireless communication module 32 are arranged on first area 9; further control circuitry, in particular power management control circuitry 33, is advantageously arranged on third area, since battery connection area 29 is, in the depicted embodiment, connected to third area 9' via a further bending area 17'. Battery connection area 29 includes battery terminals 28 and 28', and is shaped and configured to that it can be bent and arranged around a battery, such as battery 10 shown in some of the other figures, in such a way that battery terminals 28 and 28' contact the terminals of the battery.

For reasons of legibility, the connections between the various components are not depicted in Fig. 10 - and in fact, at least some of these connections, in particular microstrip 23, can be arranged on the other side of FPCB, and would therefore not necessarily be visible in such a view. If connections are indeed arranged on the other side, vias can be formed by providing an opening in FPCB 30, for instance at connection point 21.

As discussed previously, wireless communication module 32 is connected to slot antenna 19 at connection point 21. Both communication module 32 and power management circuitry 33 will need to be communicatively coupled (whether directly or indirectly) to the control circuitry of main processing unit 31; the connection between power management circuitry 33 and main processing unit 31 can, in this particular embodiment, be provided using one of bending areas 27, 27' or 27". Furthermore, since the main processing unit 31 must be able to send commands to the motor, it must be connected, either directly or indirectly, to motor connection terminals 15, 15'; this connection may for instance be provided using bending areas 17 and 24, though other solutions can also be envisaged. Power management circuitry 33 must in turn be connected to battery terminals 28 and 28'; this connection can be provided via bending area 17'.

Note that Fig. 10 is schematic; the positioning, shape and size of the various components and terminals should not be taken as representative or restrictive for the actual positioning, shape and/or size. Furthermore, while in this embodiment battery connection area 29 extends from third area 9' and motor connection area 26 extends from second area 11, it should be clear that this is in view of the particular arrangement and relative positioning of the various components in the specific device for which this FPCB is intended. For instance, in devices including a sensing unit and no drug delivery device, a motor will likely not be necessary, and motor connection area 17 may be omitted. Furthermore, while in this embodiment there is only one antenna 19, arranged on second area 11, there could be at least one additional antenna, which could be arranged on second area 11; on one of the other areas depicted in Fig. 10; or on a further area not included in this figure. An antenna array could also be considered.

By using the type of slot antenna discussed above, and providing and arranging FPCB 30 as depicted, a useful directionality was achieved for the Bluetooth signal strength. In particular, it was found that for subjects with a BMI of up to 36, sufficient signal strength for Bluetooth communication was achieved not only by positioning a smartphone between the user's legs (which is generally the most advantageous position but may be inconvenient, in particular in public) but also when the phone was held close to the user's pelvis, and even if the phone was in a user's rear pocket. Due to the particulars of this embodiment, with the antenna arranged in the left part of the device, slightly better results were found when the phone was in a right pocket, rather than a left, but this would of course be different for a different choice of arrangement, and sufficient connectivity was achieved in either pocket.

In the above, the invention has been disclosed by referring to embodiments thereof. However, it will be clear to the skilled person that the arrangement shown, while quite advantageous, it just one of many possibilities. Generally, the combination of a slot antenna arranged on an area of a flexible printed circuit board and attached to a ceramic buffer via at least one adhesive layer has been found to finally make possible what previously had not been achieved, namely robust and reliable Bluetooth connectivity within a very limited space. It should be appreciated by the person skilled in the art that various modifications are possible without deviating from the invention, the scope of which is defined by the appended claims.

## Claims

1. A vaginal drug delivery and/or vaginal sensor device (100), comprising:
a drug delivery unit comprising a reservoir (4) holding a medicament to be delivered, an outlet (6), a pump (7) for pumping said medicament out of said outlet (6), and a motor (8) for actuating said pump (7); and/or
a sensing unit comprising one or more sensors;
a flexible printed circuit board (30), FPCB, comprising a first area (9) and a second area (11);
control circuitry (31) arranged on the first area (9) of the FPCB (30), communicatively coupled to the drug delivery unit and/or sensing unit;
a wireless communication module (32) arranged on the FPCB (30), preferably configured for Bluetooth communication, wherein the wireless communication module is communicatively coupled to the control circuitry (31);
**CHARACTERIZED IN THAT** the device further comprises:
a slot antenna formed on the second area (11) of the FPCB (30) by providing a slot (19) in a metal layer (16) arranged on the second area (11);
a microstrip line (23) connecting the wireless communication module (32) to a connection point (21) at or adjacent to a first end (20) of the slot (19);
a ceramic buffer (12) arranged over the slot antenna on the second area (11); and
at least one adhesive layer (18) arranged between the ceramic buffer (12) and the slot antenna on the second area (11)
wherein multiple separate slot antennas are provided; and/or
wherein the slot (19) of the slot antenna has a first end (20) and a second end (22) and extends along a path between the first end (20) and second end (22) in such a way that the total length of the slot (19) is significantly larger than the shortest distance between the first end (20) and second end (22).

2. The device of claim 1, wherein the first area (9) and the second area (11) of the FPBC are connected via at least one bent area (24, 24', 24") of the FPCB; and/or
wherein the first area (9) is substantially planar, and wherein the first area (9) is arranged to be at an angle at least part of the second area (11), preferably to be substantially perpendicular to at least a part of the second area (11); and/or
wherein the device comprises a drug delivery unit, and wherein the motor (8) is at least in part arranged in a space delimited by the first area (9) of the FPCB and the second area (11) of the FPCB.

3. The device (100) of any of the previous claims, wherein the device (100) is deformable between an extended shape, which it assumes when little to no external force is applied thereto, and which corresponds to a substantially oval or annular ring shape extending around a central axial axis, and a collapsed shape allowing the device to be inserted into a vagina of a user.

4. The device of any of the previous claims, wherein the slot (19) of the slot antenna has a first end (20) and a second end (22) and extends along a path between the first end (20) and second end (22) in such a way that the total length of the slot (19) is significantly larger than the shortest distance between the first end (20) and second end (22) and/or
wherein the length of the slot from the connection point (21) to the end (22) furthest from the connection point is approximately an integer multiple of λ/2, with λ the wavelength of the communication signal, or an integer multiple of λ/4.

5. The device (100) of any of the preceding claims, wherein the control circuitry (31) and any other components arranged on the first area (9) are arranged on the side of the first area (9) of the FPCB (30) which faces the nearest portion of the outer surface of the device, and/or
wherein the second area (11) is substantially arranged in a first plane which is perpendicular to a central axial axis (A) of the device and/or
wherein the slot antenna is arranged on the side of the second area (11) which is oriented towards the ceramic buffer (12), or on the side of the second area (11) which is oriented away from the ceramic buffer (12) and/or
wherein the second area (11) is formed as a side flap of the FPCB (30).

6. The device (100) of any of the preceding claims, wherein the second area (11) is substantially rectangular, with two shorter sides and two longer sides, wherein a first end (20) of the slot is located close to a first one of the shorter sides, and a second end (22) of the slot (19) is located close to the other of the shorter sides,
wherein, preferably:
the second area (11) is connected to the first area (9) over at least a portion of a first one of the longer sides, or over a portion of a first one of its shorter sides, and/or
the slot (19) follows a meandering path comprising longer sections extending substantially parallel to the width of the second area (11); and shorter sections extending substantially parallel to the length of the second area (11), which connect one of the ends of adjacent longer portions, more preferably wherein the distance between two adjacent longer sections is comparable to or larger than the width of the slot (19).

7. The device (100) of any of the preceding claims,
wherein the microstrip line (23) is configured to have a resistance of about 50Ω, and/or
wherein the microstrip line (23) bridges the slot (19) close to the connection point (21) and/or
wherein a substantial section of the microstrip line (23) is arranged on the opposite side of the second area (11) of the FPCB (30) than the slot antenna, and wherein one end of the microstrip line (23) passes through an opening in the second area (11) at or close to the connection point (21).

8. The device (100) of any of the preceding claims,
wherein the ceramic buffer (12) has a first major surface which covers the slot antenna, wherein the first major surface is preferably substantially parallel to the surface of the second area (11), and/or;
wherein the ceramic buffer (12) is configured such that it substantially fills the space between the slot antenna and the nearest portion of the outer wall of the device (100), and/or;
wherein the ceramic buffer (12) is made of or comprises zirconia, and/or;
wherein the ceramic buffer (12) has a relative permittivity εᵣ between 10 and 50, preferably between 20 and 40, more preferably between 25 and 35, and/or;
wherein the at least one adhesive layer (18) has a thickness between 50 µm and 200 µm, preferably between 80 µm and 170 µm, more preferably between 90µm and 120µm.

9. The device (100) of any of the preceding claims, wherein the at least one adhesive layer (18) comprises a carrier layer between two adhesive layers, wherein the carrier layer is preferably a polyester layer, and the two adhesive layers are preferably modified acrylic adhesive layers.

10. The device (100) of any of the preceding claims, wherein the thickness and/or material properties of the at least one adhesive layer (18) are selected to achieve the desired radiation characteristics, preferably to compensate for the effect of the ceramic buffer (12), motor (8), FPCB (30) and/or or housing of the device on the operation frequency, so as to ensure that the signal emitted by the device is in the Bluetooth frequency band.

11. The device (100) of any of the preceding claims, wherein the device further comprises:
a first rigid member (101) having a first and second end;
a second rigid member (102) having a third and fourth end;
a first flexible member (111) coupled between the first and third ends;
a flexible part (110) coupled between the second and fourth ends.

12. The device (100) of claim 9, comprising a drug delivery unit, wherein:
the reservoir (4) is accommodated in the first rigid member (101);
the outlet (6) is provided in an outer wall of the second rigid member (102);
the pump (7) and motor (8) are accommodated in the second rigid member (102); and
the device (100) further includes a tube (5) which passes through the first flexible member (111) and provides a fluid communication path between the reservoir (4) and the outlet (6);
wherein the pump (7) is preferably a roller pump.

13. The device (100) of claim 11 or 12, further comprising a power source (10), such as a battery, preferably accommodated in the second rigid member (102).

14. The device (100) of claim 12 and 13, wherein the motor (8) is arranged between the power source (10) and the pump (7).

15. The device of any of claims 11-12, wherein at least one sensor is arranged in the first (101) and/or second (102) rigid member.

## Patentansprüche

1. Vaginale Arzneimittelverabreichungs- und/oder vaginale Sensorvorrichtung (100), umfassend:
eine Arzneimittelverabreichungseinheit, umfassend ein Reservoir (4), das ein zu verabreichendes Medikament enthält, einen Auslass (6), eine Pumpe (7) zum Pumpen des Medikaments aus dem Auslass (6) und einen Motor (8) zum Betätigen der Pumpe (7); und/oder
eine Erfassungseinheit, umfassend einen oder mehrere Sensoren;
eine flexible Leiterplatte (30), FPCB, umfassend einen ersten Bereich (9) und einen zweiten Bereich (11);
eine Steuerschaltung (31), die auf dem ersten Bereich (9) der FPCB (30) angeordnet, mit der Arzneimittelverabreichungseinheit und/oder Sensoreinheit kommunikativ gekoppelt ist;
ein drahtloses Kommunikationsmodul (32), das auf der FPCB (30) angeordnet, vorzugsweise für Bluetooth-Kommunikation konfiguriert ist, wobei das drahtlose Kommunikationsmodul mit dem Steuerschaltkreis (31) kommunikativ gekoppelt ist;
**DADURCH GEKENNZEICHNET, DASS** die Vorrichtung ferner umfasst:
eine Schlitzantenne, die auf dem zweiten Bereich (11) der FPCB (30) durch Bereitstellen eines Schlitzes (19) in einer Metallschicht (16), die auf dem zweiten Bereich (11) angeordnet ist, ausgebildet ist;
eine Mikrostreifenleitung (23), die das drahtlose Kommunikationsmodul (32) mit einem Verbindungspunkt (21) an einem oder benachbart zu einem Ende (20) des Schlitzes (19) verbindet;
einen Keramikpuffer (12), der über der Schlitzantenne auf dem zweiten Bereich (11) angeordnet ist; und
mindestens eine Klebeschicht (18), die zwischen dem Keramikpuffer (12) und der Schlitzantenne auf dem zweiten Bereich (11) angeordnet ist,
wobei vielfache separate Schlitzantennen bereitgestellt sind; und/oder
wobei der Schlitz (19) der Schlitzantenne ein erstes Ende (20) und ein zweites Ende (22) aufweist und sich auf derartige Weise entlang eines Pfades zwischen dem ersten Ende (20) und dem zweiten Ende (22) erstreckt, dass die Gesamtlänge des Schlitzes (19) wesentlich größer als der kürzeste Abstand zwischen dem ersten Ende (20) und dem zweiten Ende (22) ist.

2. Vorrichtung nach Anspruch 1, wobei der erste Bereich (9) und der zweite Bereich (11) der FPBC über mindestens einen gebogenen Bereich (24, 24', 24") der FPCB verbunden sind; und/oder
wobei der erste Bereich (9) im Wesentlichen planar ist, und wobei der erste Bereich (9) in einem Winkel zu mindestens einem Teil des zweiten Bereichs (11) angeordnet ist, vorzugsweise um im Wesentlichen senkrecht zu mindestens einem Teil des zweiten Bereichs (11) zu stehen; und/oder
wobei die Vorrichtung eine Arzneimittelverabreichungseinheit umfasst, und wobei der Motor (8) mindestens teilweise in einem Raum, der durch den ersten Bereich (9) der FPCB und den zweiten Bereich (11) der FPCB begrenzt ist, angeordnet ist.

3. Vorrichtung (100) nach einem der vorstehenden Ansprüche, wobei die Vorrichtung (100) zwischen einer ausgedehnten Form, die sie annimmt, wenn wenig bis keine äußere Kraft darauf ausgeübt wird und die einer im Wesentlichen ovalen oder ringförmigen Ringform entspricht, die sich um eine mittlere axiale Achse erstreckt, und einer kollabierten Form, die es ermöglicht, die Vorrichtung in eine Vagina einer Benutzerin einzuführen, verformbar ist.

4. Vorrichtung nach einem der vorstehenden Ansprüche, wobei der Schlitz (19) der Schlitzantenne ein erstes Ende (20) und ein zweites Ende (22) aufweist und sich entlang eines Pfades zwischen dem ersten Ende (20) und dem zweiten Ende (22) auf derartige Weise erstreckt, dass die Gesamtlänge des Schlitzes (19) wesentlich größer als der kürzeste Abstand zwischen dem ersten Ende (20) und dem zweiten Ende (22) ist und/oder
wobei die Länge des Schlitzes von dem Verbindungspunkt (21) bis zu dem Ende (22), das am weitesten von dem Verbindungspunkt entfernt ist, etwa ein ganzzahliges Vielfaches von λ/2, wobei λ die Wellenlänge des Kommunikationssignals ist, oder ein ganzzahliges Vielfaches von λ/4 beträgt.

5. Vorrichtung (100) nach einem der vorstehenden Ansprüche, wobei der Steuerschaltkreis (31) und jegliche anderen Komponenten, die auf dem ersten Bereich (9) angeordnet sind, auf der Seite des ersten Bereichs (9) der FPCB (30) angeordnet sind, die dem nächstgelegenen Abschnitt der Außenoberfläche der Vorrichtung zugewandt ist, und/oder
wobei der zweite Bereich (11) im Wesentlichen in einer ersten Ebene angeordnet ist, die senkrecht zu einer mittleren axialen Achse (A) der Vorrichtung ist, und/oder
wobei die Schlitzantenne auf der Seite des zweiten Bereichs (11), der zu dem Keramikpuffer (12) hin ausgerichtet ist oder auf der Seite des zweiten Bereichs (11), der von dem Keramikpuffer (12) weg ausgerichtet ist, angeordnet ist und/oder
wobei der zweite Bereich (11) als eine Seitenklappe der FPCB (30) ausgebildet ist.

6. Vorrichtung (100) nach einem der vorstehenden Ansprüche, wobei der zweite Bereich (11) im Wesentlichen rechteckig ist, mit zwei kürzeren Seiten und zwei längeren Seiten, wobei sich ein erstes Ende (20) des Schlitzes nahe einer ersten der kürzeren Seiten befindet und sich ein zweites Ende (22) des Schlitzes (19) nahe der anderen der kürzeren Seiten befindet,
wobei vorzugsweise:
der zweite Bereich (11) mit dem ersten Bereich (9) über mindestens einen Abschnitt einer ersten der längeren Seiten oder über einen Abschnitt einer ersten seiner kürzeren Seiten verbunden ist, und/oder
der Schlitz (19) einem mäanderförmigen Pfad, umfassend längere Anteile, die sich im Wesentlichen parallel zu der Breite des zweiten Bereichs (11) erstrecken, folgt; und kürzere Anteile, die sich im Wesentlichen parallel zu der Länge des zweiten Bereichs (11) erstrecken, die eines der Enden benachbarter längerer Abschnitte verbinden, mehr bevorzugt wobei der Abstand zwischen zwei benachbarten längeren Anteilen vergleichbar mit oder größer als die Breite des Schlitzes (19) ist.

7. Vorrichtung (100) nach einem der vorstehenden Ansprüche,
wobei die Mikrostreifenleitung (23) konfiguriert ist, um einen Widerstand von etwa 50 Ω aufzuweisen, und/oder
wobei die Mikrostreifenleitung (23) den Schlitz (19) nahe des Verbindungspunkts (21) überbrückt und/oder
wobei ein wesentlicher Anteil der Mikrostreifenleitung (23) auf der gegenüberliegenden Seite des zweiten Bereichs (11) der FPCB (30) zu der Schlitzantenne angeordnet ist, und wobei ein Ende der Mikrostreifenleitung (23) durch eine Öffnung in dem zweiten Bereich (11) an oder nahe dem Verbindungspunkt (21) verläuft.

8. Vorrichtung (100) nach einem der vorstehenden Ansprüche,
wobei der Keramikpuffer (12) eine erste Hauptoberfläche aufweist, die die Schlitzantenne abdeckt, wobei die erste Hauptoberfläche vorzugsweise im Wesentlichen parallel zu der Oberfläche des zweiten Bereichs (11) ist, und/oder;
wobei der Keramikpuffer (12) derart konfiguriert ist, dass er den Raum zwischen der Schlitzantenne und dem nächstgelegenen Abschnitt der Außenwand der Vorrichtung (100) im Wesentlichen ausfüllt, und/oder;
wobei der Keramikpuffer (12) aus Zirkonoxid gefertigt ist oder dieses umfasst, und/oder;
wobei der Keramikpuffer (12) eine relative Permittivität εᵣ zwischen 10 und 50, vorzugsweise zwischen 20 und 40, mehr bevorzugt zwischen 25 und 35, aufweist und/oder;
wobei die mindestens eine Klebeschicht (18) eine Dicke zwischen 50 µm und 200 µm, vorzugsweise zwischen 80 µm und 170 µm, mehr bevorzugt zwischen 90 µm und 120 µm aufweist.

9. Vorrichtung (100) nach einem der vorstehenden Ansprüche, wobei die mindestens eine Klebeschicht (18) eine Trägerschicht zwischen zwei Klebeschichten umfasst, wobei die Trägerschicht vorzugsweise eine Polyesterschicht ist und die zwei Klebeschichten vorzugsweise modifizierte Acrylklebeschichten sind.

10. Vorrichtung (100) nach einem der vorstehenden Ansprüche, wobei die Dicke und/oder Materialeigenschaften der mindestens einen Klebeschicht (18) ausgewählt sind, um die gewünschten Strahlungseigenschaften zu erreichen, vorzugsweise um die Auswirkung des Keramikpuffers (12), des Motors (8), der FPCB (30) und/oder des Gehäuses der Vorrichtung auf die Betriebsfrequenz auszugleichen, um sicherzustellen, dass das Signal, das durch die Vorrichtung emittiert wird, in dem Bluetooth-Frequenzband liegt.

11. Vorrichtung (100) nach einem der vorstehenden Ansprüche, wobei die Vorrichtung ferner umfasst:
ein erstes starres Element (101), das ein erstes und ein zweites Ende aufweist;
ein zweites starres Element (102), das ein drittes und ein viertes Ende aufweist;
ein erstes flexibles Element (111), das zwischen dem ersten und dem dritten Ende gekoppelt ist;
ein flexibles Teil (110), das zwischen dem zweiten und dem vierten Ende gekoppelt ist.

12. Vorrichtung (100) nach Anspruch 9, umfassend eine Arzneimittelverabreichungseinheit, wobei:
das Reservoir (4) in dem ersten starren Element (101) untergebracht ist;
der Auslass (6) in einer Außenwand des zweiten starren Elements (102) bereitgestellt ist;
die Pumpe (7) und der Motor (8) in dem zweiten starren Element (102) untergebracht sind; und
die Vorrichtung (100) ferner einen Schlauch (5) einschließt, der durch das erste flexible Element (111) verläuft und einen Fluidverbindungsweg zwischen dem Reservoir (4) und dem Auslass (6) bereitstellt;
wobei die Pumpe (7) vorzugsweise eine Rollenpumpe ist.

13. Vorrichtung (100) nach Anspruch 11 oder 12, ferner umfassend eine Energiequelle (10), wie eine Batterie, die vorzugsweise in dem zweiten starren Element (102) untergebracht ist.

14. Vorrichtung (100) nach Anspruch 12 und 13, wobei der Motor (8) zwischen der Energiequelle (10) und der Pumpe (7) angeordnet ist.

15. Vorrichtung nach einem der Ansprüche 11 bis 12, wobei mindestens ein Sensor in dem ersten (101) und/oder zweiten (102) starren Element angeordnet ist.

## Revendications

1. Dispositif (100) d'administration de médicament par voie vaginale et/ou formant capteur vaginal, comprenant :
une unité d'administration de médicament comprenant un récipient (4) contenant un médicament à administrer, une sortie (6), une pompe (7) pour le pompage dudit médicament hors de ladite sortie (6), et un moteur (8) pour l'actionnement de ladite pompe (7) ; et/ou
une unité de détection comprenant un ou plusieurs capteurs ;
une carte de circuit imprimé flexible (30), FPCB, comprenant une première zone (9) et une seconde zone (11) ;
une circuiterie de commande (31) agencée au niveau de la première zone (9) de la FPCB (30), couplée en communication à l'unité d'administration de médicament et/ou à l'unité de détection ;
un module de communication sans fil (32) agencé sur la FPCB (30), de préférence configuré pour une communication Bluetooth, dans lequel le module de communication sans fil est couplé en communication à la circuiterie de commande (31) ;
**caractérisé en ce que** le dispositif comprend en outre :
une antenne à fente formée au niveau de la seconde zone (11) de la FPCB (30) en fournissant une fente (19) dans une couche métallique (16) agencée au niveau de la seconde zone (11) ;
une ligne microruban (23) connectant le module de communication sans fil (32) à un point de connexion (21) au niveau d'une première extrémité (20) de la fente (19) ou adjacent à celle-ci ;
un tampon céramique (12) agencé sur l'antenne à fente au niveau de la seconde zone (11) ; et
au moins une couche adhésive (18) agencée entre le tampon céramique (12) et l'antenne à fente au niveau de la seconde zone (11)
dans lequel plusieurs antennes à fentes séparées sont fournies ; et/ou
dans lequel la fente (19) de l'antenne à fente a une première extrémité (20) et une seconde extrémité (22) et s'étend le long d'un trajet entre la première extrémité (20) et la seconde extrémité (22) de manière telle que la longueur totale de la fente (19) est significativement plus grande que la distance la plus courte entre la première extrémité (20) et la seconde extrémité (22).

2. Dispositif selon la revendication 1, dans lequel la première zone (9) et la seconde zone (11) de la FPBC sont reliées par l'intermédiaire d'au moins une zone courbée (24, 24', 24") de la FPCB ; et/ou
dans lequel la première zone (9) est sensiblement plane, et dans lequel la première zone (9) est agencée de manière à être à un certain angle par rapport à au moins une partie de la seconde zone (11), de préférence de manière à être sensiblement perpendiculaire à au moins une partie de la seconde zone (11) ; et/ou
dans lequel le dispositif comprend une unité d'administration de médicament, et dans lequel le moteur (8) est au moins en partie agencé dans un espace délimité par la première zone (9) de la FPCB et la seconde zone (11) de la FPCB.

3. Dispositif (100) selon l'une quelconque des revendications précédentes, dans lequel le dispositif (100) est déformable entre une forme étendue, qu'il prend lorsque peu ou pas de force extérieure est appliquée sur celui-ci, et qui correspond à une forme d'anneau sensiblement ovale ou annulaire s'étendant autour d'un axe axial central, et une forme rétractée permettant au dispositif d'être inséré dans le vagin d'une utilisatrice.

4. Dispositif selon l'une quelconque des revendications précédentes, dans lequel la fente (19) de l'antenne à fente a une première extrémité (20) et une seconde extrémité (22) et s'étend le long d'un trajet entre la première extrémité (20) et la seconde extrémité (22) de manière telle que la longueur totale de la fente (19) est significativement plus grande que la distance la plus courte entre la première extrémité (20) et la seconde extrémité (22) et/ou
dans lequel la longueur de la fente depuis le point de connexion (21) jusqu'à l'extrémité (22) la plus éloignée du point de connexion est approximativement un multiple entier de λ/2, λ représentant la longueur d'onde du signal de communication, ou un multiple entier de λ/4.

5. Dispositif (100) selon l'une quelconque des revendications précédentes, dans lequel la circuiterie de commande (31) et tout autre composant quelconque agencé au niveau de la première zone (9) sont agencés sur le côté de la première zone (9) de la FPCB (30) qui fait face à la partie la plus proche de la surface extérieure du dispositif et/ou
dans lequel la seconde zone (11) est sensiblement agencée dans un premier plan qui est perpendiculaire à un axe axial central (A) du dispositif et/ou
dans lequel l'antenne à fente est agencée sur le côté de la seconde zone (11) qui est orienté vers le tampon céramique (12), ou sur le côté de la seconde zone (11) qui est orienté à l'écart du tampon céramique (12), et/ou
dans lequel la seconde zone (11) est formée en tant que rabat latéral de la FPCB (30).

6. Dispositif (100) selon l'une quelconque des revendications précédentes, dans lequel la seconde zone (11) est sensiblement rectangulaire, avec deux côtés plus courts et deux côtés plus longs, dans lequel une première extrémité (20) de la fente est située à proximité d'un premier côté parmi les côtés plus courts, et une seconde extrémité (22) de la fente (19) est située à proximité de l'autre côté parmi les côtés plus courts,
dans lequel, de préférence :
la seconde zone (11) est reliée à la première zone (9) sur au moins une partie d'un premier côté parmi les côtés plus longs, ou sur une partie d'un premier côté parmi ses côtés plus courts, et/ou
la fente (19) suit un chemin sinueux comprenant des sections plus longues s'étendant sensiblement parallèlement à la largeur de la seconde zone (11) ; et des sections plus courtes s'étendant sensiblement parallèlement à la longueur de la seconde zone (11), qui relient l'une des extrémités de parties plus longues adjacentes, plus préférablement dans lequel la distance entre deux sections plus longues adjacentes est comparable ou supérieure à la largeur de la fente (19).

7. Dispositif (100) selon l'une quelconque des revendications précédentes,
dans lequel la ligne microruban (23) est conçue pour avoir une résistance d'environ 50 Ω et/ou
dans lequel la ligne microruban (23) recouvre la fente (19) à proximité du point de connexion (21) et/ou
dans lequel une section importante de la ligne microruban (23) est agencée sur le côté opposé de la seconde zone (11) de la FPCB (30) par rapport à l'antenne à fente, et dans lequel une extrémité de la ligne microruban (23) passe à travers une ouverture dans la seconde zone (11) au niveau ou à proximité du point de connexion (21).

8. Dispositif (100) selon l'une quelconque des revendications précédentes,
dans lequel le tampon céramique (12) a une première surface principale qui recouvre l'antenne à fente, dans lequel la première surface principale est de préférence sensiblement parallèle à la surface de la seconde zone (11), et/ou ;
dans lequel le tampon céramique (12) est conçu de telle sorte qu'il remplit sensiblement l'espace entre l'antenne à fente et la partie la plus proche de la paroi extérieure du dispositif (100), et/ou ;
dans lequel le tampon céramique (12) est constitué de zircone ou en comprend, et/ou ;
dans lequel le tampon céramique (12) a une permittivité relative εᵣ comprise entre 10 et 50, de préférence entre 20 et 40, plus préférablement entre 25 et 35, et/ou ;
dans lequel l'au moins une couche adhésive (18) a une épaisseur comprise entre 50 µm et 200 µm, de préférence entre 80 µm et 170 µm, plus préférablement entre 90 µm et 120 µm.

9. Dispositif (100) selon l'une quelconque des revendications précédentes, dans lequel l'au moins une couche adhésive (18) comprend une couche de support entre deux couches adhésives, dans lequel la couche de support est de préférence une couche de polyester, et les deux couches adhésives sont de préférence des couches d'adhésif acrylique modifié.

10. Dispositif (100) selon l'une quelconque des revendications précédentes, dans lequel l'épaisseur et/ou les propriétés de matériau de l'au moins une couche adhésive (18) sont choisies pour obtenir les caractéristiques de rayonnement souhaitées, de préférence pour compenser l'effet du tampon céramique (12), du moteur (8), de la FPCB (30) et/ou ou du boîtier du dispositif sur la fréquence de fonctionnement, de manière à s'assurer que le signal émis par le dispositif est situé dans la bande de fréquences Bluetooth.

11. Dispositif (100) selon l'une quelconque des revendications précédentes, dans lequel le dispositif comprend en outre :
un premier élément rigide (101) ayant une première et une deuxième extrémité ;
un second élément rigide (102) ayant une troisième et une quatrième extrémité ;
un premier élément flexible (111) accouplé entre la première et la troisième extrémité ;
une partie flexible (110) accouplée entre la deuxième et la quatrième extrémité.

12. Dispositif (100) selon la revendication 9, comprenant une unité d'administration de médicament, dans lequel :
le récipient (4) est logé dans le premier élément rigide (101) ;
la sortie (6) est prévue dans une paroi extérieure du second élément rigide (102) ;
la pompe (7) et le moteur (8) sont logés dans le second élément rigide (102) ; et
le dispositif (100) comporte en outre un tube (5) qui passe à travers le premier élément flexible (111) et fournit une voie de communication fluidique entre le récipient (4) et la sortie (6) ;
dans lequel la pompe (7) est de préférence une pompe à galets.

13. Dispositif (100) selon la revendication 11 ou 12, comprenant en outre une source d'alimentation (10), telle qu'une batterie, de préférence logée dans le second élément rigide (102).

14. Dispositif (100) selon les revendications 12 et 13, dans lequel le moteur (8) est agencé entre la source d'alimentation (10) et la pompe (7).

15. Dispositif selon l'une quelconque des revendications 11 à 12, dans lequel au moins un capteur est agencé dans le premier (101) et/ou le second (102) élément rigide.
